# NEUE EUROPÄISCHE PATENTSCHRIFT

(11) **EP 0 943 317 B2**
(45) Veröffentlichungstag und Bekanntmachung des Hinweises auf die Entscheidung über den Einspruch: **04.01.2012**
(45) Hinweis auf die Patenterteilung: 11.04.2001
(21) Anmeldenummer: 99101159.4
(22) Anmeldetag: 22.01.1999
(51) Int. Cl.: A61K 8/37, A61Q 5/10

(54) **Viskostätstabilisierung von Wässrigen Haarfärbeemulsionen**
Stabilizing the viscosity of hair dyeing emulsions
Stabilisation de la viscosité d'emulsions aqueuses de tinture capillaire

(30) Priorität: 21.02.1998 DE 19807510
(43) Veröffentlichungstag der Anmeldung: 22.09.1999
(73) Patentinhaber: KPSS-Kao Professional Salon Services GmbH, 64297 Darmstadt (DE)
(72) Erfinder: Kufner, Frank, 64297 Darmstadt (DE); Lorenz, Heribert, 64401 Gross-Bieberau (DE)

(56) Entgegenhaltungen:
- EP-A- 0 193 471
- EP-A- 0 885 607
- WO-A-86/02546
- DE-A- 4 405 510
- JP-A- 9 169 623

## Beschreibung

Die vorliegende Erfindung betrifft Verwendung von Fettsäuretriglycerids zur Viskositätstabilisierung von wäßrigen Haarfärbeemulsionen, enthaltend mindestens ein Oxidationsfarbstoff-Vorprodukt.

Permanente Haarfärbemittel auf Basis von Oxidationsfarbstoffen erfreuen sich einer weiten Verbreitung. Ihre Anwendung erfolgt in der Regel dergestalt, daß eine flüssige , zumeist als wäßrige Emulsion vorliegende, mindestens ein Oxidationsfarbstoff-Vorprodukt, im allgemeinen mindestens eine Entwickler- und mindestens eine Kupplersubstanz, enthaltende Zusammensetzung unmittelbar vor der Anwendung mit einer Peroxid enthaltenden Zusammensetzung vermischt, und die Mischung auf das Haar aufgebracht wird.

Dabei ist die Einhaltung gewisser Viskositätsverhältnisse zweckmäßig. Es hat sich jedoch gezeigt, daß diese Emulsionen häufig thixotrope Eigenschaften aufweisen, d.h., bei der Lagerung in der Dose oder Tube ihre Viskosität erhöhen, was dann bei der Applikation und der Vermischung mit der Peroxid-Zusammensetzung Probleme bereitet.

Die Erfindung geht daher von der Aufgabenstellung aus, diese Probleme zu vermeiden und Haarfärbeemulsionen mit stabiler Viskosität zur Verfügung zu stellen.

Erfindungsgemäß wurde festgestellt, daß eine stabile Viskosität wäßriger HaarfärbeEmulsionen, die mindestens ein Oxidationsfarbstoff-Vorprodukt enthalten, dann erreicht wird wenn sie 0,5 bis 10, vorzugsweise etwa 1 bis 5, insbesondere etwa 1,5 bis 2,5 Gew.-%, berechnet auf die Gesamtzusammensetzung der Emulsion, mindestens eines C₆-C₁₈-Fettsäuretriglycerids enthalten.

Als Fettsäuretriglycerid wird Caprinsäuretriglycerid bevorzugt, jedoch sind grundsätzlich auch andere Triglyceride verwendbar, z.B,. jene von Caprylsäure, Laurinsäure, Myristinsäure oder Palmitinsäure sowie solche von Mischsäuren wie Pflanzenfettsäuren, z.B. Kokosölfettsäuren.

Die sonstigen möglichen Bestandteile der erfindungsgemäßen Haarfärbemittel sind an sich bekannt.

Diese können üblicherweise weitere Emulgatoren und Tenside, Lösungsvermittler, Verdickungsmittel, haarkonditionierende Substanzen wie Eiweißhydrolysate und synthetische Polymere, Stabilisatoren, Verdünnungsmittel, etc. enthalten.

Ausführliche Angaben über die Zusammensetzung und Herstellung von Haarfärbemitteln und geeignete Oxidationsfarbstoff-Vorprodukte finden sich beispielsweise in der Monographie von K. Schrader, Grundlagen und Rezepturen der Kosmetika (1989, 2. Auflage, Dr. A. Hüthig Verlag), S. 782-815, auf die hier ausdrücklich Bezug genommen wird.

Die Anwendung der Oxidationsfarbstoff-Vorprodukte enthaltenden Haarfärbemittel erfolgt durch Vermischen mit Peroxiden, insbesondere Wasserstoffperoxidlösung oder -lotion, unmittelbar vor der Applikation auf das Haar.

Die auf das Haar aufzubringende, gebrauchsfertige Färbemischung weist dabei vorzugsweise einen alkalischen pH-Wert im Bereich von 7,5 bis 9,5 auf, jedoch sind auch neutrale und schwach saure Endprodukte geeignet, z.B. im Bereich von etwa 5,5 bis etwa 7,5.

Im folgenden wird anhand von zwei Ausführungsbeispielen die Wirkung der erfindungsgemäßen Zusammensetzungen beschrieben.

| | A | B |
|---|---|---|
| Cetylstearylalkohol | 10,00 | 8,00 |
| Stearinsäuremonoethanolamid | 2,00 | 2,00 |
| Kokossäuremonoethanolamid | 2,00 | 2,00 |
| Ölsäure | 1,00 | 1,00 |
| Caprinsäuretriglycerid | - | 2,00 |
| Hydroxyethlcellulose | 0,50 | 0,50 |
| Natriumlaurylsulfat | 0,50 | 0,50 |
| Tetranatrium-EDTA | 0,20 | 0,20 |
| Ammoniumchlorid | 0,25 | 0,25 |
| Natriumsulfit | 0,50 | 0,50 |
| Natriumhydroxid | 0,48 | 0,48 |
| Mangandioxid | 0,12 | 0,12 |
| Proteinhydrolysat | 1,00 | 1,00 |
| Parfum | 0,20 | 0,20 |
| 2,5-Diaminotoluolsulfat | 0,22 | 0,22 |
| 4-Aminophenol | 0,10 | 0,10 |
| 2-Methylresorcin | 0,05 | 0,05 |
| 3-Aminophenol | 0,05 | 0,05 |
| 2,5,6-Triamino-4-hydroxypyrimidinsulfat | 0,10 | 0,10 |
| Wasser ad | 100,00 | 100,00 Gew.-% |

Die Viskosität der Zusammensetzungen A und B wurde nach der Herstellung sowie nach 3, 5, 9, 12 und 16 Wochen Lagerung bei Raumtemperatur im Rotationsviskosimeter "Physica Rheolab MC 100" mit dem Meßsystem PP32/1,10 mm bei 20°C bestimmt.

Die angegebenen Werte stellen mpa.s dar.

### Ergebnis:

| **Wochen** | **Zusammensetzung A** | **Zusammensetzung B** |
|---|---|---|
| 0 | 4600 | 5100 |
| 3 | 5500 | 5200 |
| 5 | 6800 | 5100 |
| 9 | 8100 | 5100 |
| 12 | 9000 | 5200 |
| 16 | 9600 | 5100 |

Während die Viskosität der erfindungsgemäßen Zusammensetzung B über 4 Monate stabil blieb, stieg die Viskosität der bekannten Zusammensetzung A kontinuierlich an.

## Patentansprüche

1. Verwendung mindestens eines C₆-C₁₈-Fettsäuretriglycerids zur Viskositätstabilisierung von wäßrigen Haarfärbeemulsionen, die mindestens ein Oxidationsfarbstoff-Vorprodukt enthalten.

2. Verwendung nach Anspruch 1 **dadurch gekennzeichnet, daß** die Emulsion 0,5 bis 10 Gew.-%, berechnet auf die Gesamtzusammensetzung, mindestens eines C₆-C₁₈-Fettsäuretriglycerids enthält.

3. Verwendung nach Ansprüche 1 und 2, **dadurch gekennzeichnet, daß** es als Fettsäuretriglycerid Caprinsäuretriglycerid enthält.

## Claims

1. Use of at least one C₆ - C₁₈ -fatty acid triglyceride to stabilize the viscosity of aqueous hair dyeing emulsions comprising at least one hair dyestuff precursor.

2. Use according to claim 1 **characterized in that** the emulsion comprises 0.5 to 10% by weight, calculated to the total composition, of at least one C₆ - C₁₈ -fatty acid triglyceride.

3. Use according to claims 1 and 2 **characterized in that** it comprises capric acid triglyceride as fatty acid triglyceride.

## Revendications

1. Utilisation d'au moins un triglyceride d'acide gras en C₆ - C₁₈ pour stabilizer la viscosité d'émulsion aqueuses de teinture capillaire contenant au moins un precursor de colorant d'oxidation.

2. Utilisation selon la revendication 1 **caractérisée en ce que** l'émulsion continent 0,5 à 10% en poids, rapporté à la composition totale, d'au moins un triglyceride d'acide gras en C₆ - C₁₈.

3. Utilisation selon la revendication 1 et 2 **caractérisée en ce qu'**elle continent, comme triglyceride d'acide gras, un triglyceride d'acide caprique.
